## Europäisches Patentamt

## European Patent Office

## Office européen des brevets

(11) Publication number: **0 016 380**

**A1**

(12) # EUROPEAN PATENT APPLICATION

(21) Application number: **80101142.0**

(22) Date of filing: **06.03.80**

(51) Int. Cl.³: **C 07 J 9/00**
**C 07 J 53/00**

(30) Priority: **15.03.79 US 20896**

(43) Date of publication of application:
**01.10.80 Bulletin 80 20**

(84) Designated Contracting States:
**DE FR GB NL**

(71) Applicant: **EASTMAN KODAK COMPANY**
**343 State Street**
**Rochester, New York 14650(US)**

(72) Inventor: **Foster, Charles Howard**
**P.O. Box 511**
**Kingsport Tennessee(US)**

(74) Representative: **Brandes, Jürgen, Dipl.-Chem. Dr. et al,**
**Thierschstrasse 8**
**D-8000 München 22(DE)**

(54) Separation of stigmasterol from phytosterols.

(57) A mixture of phytosterols containing stigmasterol, sitosterol and campesterol is
 a) converted to the i-methyl ethers of the phytosterols,
 b) the i-methyl ethers are ozonized,
 c) the ozonide is reduced to form a mixture of 6-ß-methoxy-3α, 5α-cyclodinorcholanaldehyde, sitosterol i-methyl ether and campesterol i-methyl ether and,
 d) the 6-ß-methoxy-3α, 5α-cyclodinoranaldehyde is separated from the mixture.

EP 0 016 380 A1

-1-

## SEPARATION OF STIGMASTEROL FROM PHYTOSTEROLS

This invention relates to the separation of stigmasterol from phytosterols containing stigmasterol, sitosterol and campesterol. The phytosterols are reacted to form the corresponding phytosterol i-methyl ethers. The phytosterol i-methyl ethers are ozonized to form the aldehyde of the i-methyl ether of stigmasterol, 6-8-methoxy-3α,5α-cyclodinorcholanaldehyde, which is separated from the other phytosterol i-methyl ethers.

The naturally occurring phytosterol components of vegetable oils are composed of mixtures of phytosterols which have similar chemical and physical properties, making separation of the phytosterols into individual components difficult. For example, oils such as soybean oil contain phytosterols which are mixtures of about 25 percent stigmasterol and about 75 percent sitosterol and campesterol. The stigmasterol is extremely difficult to separate from the sitosterol because the two compounds differ structurally by a single double bond in the aliphatic side chain. The separation of stigmasterol from sitosterol and the other phytosterols such as campesterol is important because stigmasterol can be used in the preparation of pharmaceuticals such as progesterone.

Prior to this invention, one method for the separation of stigmasterol from phytosterol mixtures was carried out by the Winders and Hauth bromination method whereby a mixture of phytosterol acetates is brominated, and the relatively insoluble stigmasterol acetate tetrabromide is crystallized. However, this bromination procedure is unsatisfactory for large scale commercial processes for the separation of stigmasterol because it is a relatively expensive process, and the yields are reported to be low.

Other processes, such as extraction or

-2-

leaching processes, have also been developed and used in commercial processes for separation of stigmasterol. These extraction or leaching processes involve counter-current extraction or leaching employing large amounts of different solvents. Extensive processing steps, expensive equipment and substantial amounts of labor and energy to isolate stigmasterol are required. The process of this invention is a relatively simple, inexpensive and less energy and labor consuming process useful commercially to separate stigmasterol from other phytosterols.

In accordance with this invention a mixture of phytosterols containing stigmasterol, sitosterol and campesterol is

    a)    converted to the i-methyl ethers of the phytosterols,

    b)    the i-methyl ethers are ozonized,

    c)    the ozonide is reduced to form a mixture of 6-$\beta$-methoxy-3$\alpha$,5$\alpha$-cyclodinorchol-analdehyde, sitosterol i-methyl ether and campesterol i-methyl ether and,

    d)    the 6-$\beta$-methoxy-3$\alpha$,5$\alpha$-cyclodinoran-aldehyde is separated from the mixture.

The specific example provides a preferred procedure for carrying out the process of this invention. The mixture of i-methyl ethers of the phytosterols can be ozonized with ozone or a mixture of ozone and oxygen at a temperature of -80$^{\circ}$C. to 0$^{\circ}$C. in a solvent such as a 3:1 to 1:3 methylene dichloride/methyl alcohol solvent. The ozonide can be reduced with trimethyl phosphite or other reducing agent and the mixture can be washed with aqueous sodium sulfite, then washed with water, and the product subsequently dried over sodium sulfate. The solvent can then be evaporated to provide a yellow oil containing a mixture of 6-$\beta$-methoxy-3$\alpha$,5$\alpha$-cyclodinorcholanaldehyde

and sitosterol i-methyl ether and campesterol i-methyl ether. The 6-β-methoxy-3α,5α-cyclodinorcholanaldehyde can be separated from this mixture by chromatography or by preparing the bisulfite adduct. The 6-β-methoxy-3α,5α-cyclodinorcholanaldehyde can be used as a starting material to prepare other steroids such as progesterone, for example.

The ozonide can be reduced by other reducing agents in place of the trimethyl phosphite. For example, other reducing agents such as the combination of zinc and acetic acid, sodium bisulfite, dimethyl sulfide, or formaldehyde can also be used.

EXAMPLE

Preparation of i-methyl ethers of soy sterols. To 300 g. of mixed soy sterols (about 20% stigmasterol) in 2000 ml of pyridine was added 277 g. of p-toluenesulfonyl chloride. After stirring at room temperature 12-24 hrs, the p-toluenesulfonate esters of the sterols were isolated by pouring the reaction mixture into aqueous $NaHCO_3$ and filtering off the white solid. The sulfonates were washed with water and dried (isolated 415 g.).

48 g. of the p-toluenesulfonate esters of mixed phytosterols were placed in a mixture of 48 g of potassium acetate in 2400 ml of methanol and heated at reflux 8-24 hrs. Dilution with water, extraction with pentane, drying over $Na_2SO_4$, and evaporating gave the mixed i-methyl ethers of soy sterols (30.4 g.)

The mixture of i-methyl ethers of soy sterols was then treated with ozone by passing a stream of ozone/oxygen through a solution of the mixture of sterol i-methyl ethers in 250 ml of $CH_2Cl_2$/MeOH (3:1) at -65 to -75°C. until the blue color of ozone persisted. The mixture was flushed with nitrogen to remove excess ozone, then treated with 7.5 ml of trimethyl phosphite and allowed to warm to room temperature. After washing with $Na_2SO_3$ followed by washing with water, drying over $MgSO_4$ and evaporation of volatiles, an oil was obtained which contained 6-β-methoxy-3α,5α-cyclodinorcholanaldehyde and the i-methyl ethers of sitosterol and campesterol.

The 6-β-methoxy-3α,5α-cyclodinorcholanaldehyde can be separated from the i-methyl ethers of sitosterol and campesterol in the resulting oil by chromatography. To a column (36.8 cm x 5 cm) packed with Doucil (a sodium alumino silicate base exchange material) was added 28 g of the oil recovered above in 100 ml of heptane. Elution of sitosterol i-methyl ether and campesterol i-methyl ether with heptane (1250 ml) followed by elution of 6-β-methoxy-3α,5α-cyclodinorcholanaldehyde with 30% acetone in heptane gave 5.75 g of oil which was essentially pure 6-β-methoxy-3α,5α-cyclodinorcholanaldehyde. The first fraction contained sitosterol i-methyl ether, campesterol i-methyl ether and some 6-β-methoxy-3α,5α-cyclodinorcholanaldehyde.

The 6-β-methoxy-3α,5α-cyclodinorchloranaldehyde can also be separated from the i-methyl ethers of sitosterol and campesterol in the resulting oil by formation of the bisulfite adduct.

The resulting oil (sitosterol i-methyl ether, campesterol i-methyl ether and 6-β-methoxy-3α,5α-cyclodinorcholanaldehyde) (29.0 g) was mixed with ethyl alcohol (200 ml). A saturated aqueous sodium bisulfite solution (250 ml) was added and the mixture was refluxed 1 hour. The mixture was cooled to $10^{\circ}$C. and a white solid was isolated by filtration. The solid was washed with toluene. Evaporation of toluene gave 19.0 g of oil which was sitosterol i-methyl ether and campesterol i-methyl ether. The original ethyl alcohol/water/-sodium bisulfite filtrate was extracted with toluene.

Evaporation of the toluene layer gave 6-β-methoxy-3α,5α-cyclodinorcholanaldehyde as a viscous oil (7.5 g).

-1-

Claims:

1. The process for separating stigmasterol from a mixture of phytosterols containing stigmasterol, sitosterol and campesterol characterized by

a)     converting the phytosterols to the i-methyl ethers of the phytosterols,

b)     ozonizing the i-methyl ethers of the phytosterols,

c)     reducing the ozonide to form a mixture of 6-β-methoxy-3α,5α-cyclodinorcholanaldehyde, sitosterol i-methyl ether and campesterol i-methyl ether, and

d)     separating 6-β-methoxy-3α,5α-cyclodinorcholanaldehyde from the mixture.

2. The process according to Claim 1 wherein the aldehyde is separated by chromatography.

3. The process according to Claim 1 wherein the aldehyde is separated by forming a bisulfite adduct.

0016380

<br>

European Patent Office

**EUROPEAN SEARCH REPORT**

Application number

EP 80 10 1142

| | DOCUMENTS CONSIDERED TO BE RELEVANT | | CLASSIFICATION OF THE APPLICATION (Int. Cl. ³) |
|---|---|---|---|
| Category | Citation of document with indication, where appropriate, of relevant passages | Relevant to claim | |
| | STEROIDS, vol. 15 (1), January 1970, San Francisco, US R.F.N. HUTCHINS et al. "The synthesis and the mass and nuclear magnetic resonance spectra of side chain isomers of cholesta-5,22-dien-3β-ol and cholesta-5,22,24-trien-3β ol". pages 113-130. <br> * pages 116,125,126 * | 1 | C 07 J 9/00 <br> 53/00 |
| A | BERICHTE DER DEUTSCHEN CHEMISCHEN GESELLSCHAFT, vol. 39 (4), 1906, Berlin, DE A. WINDAUS et al. "Uber Stigmasterin ein neues Phytosterin aus Calabar-Bohnen". pages 4378-4384. <br> * Whole article * | 1 | |
| A | US - A - 2 839 544 (J.W. GREINER) <br> * Claim 1 * | 1 | |
| A | US - A - 2 520 143 (E.B. HERSHBERG) <br> * Claim 1 * | 1 | |

**TECHNICAL FIELDS SEARCHED (Int.Cl. ³)**

C 07 J 9/00
53/00

**CATEGORY OF CITED DOCUMENTS**

X: particularly relevant
A: technological background
O: non-written disclosure
P: intermediate document
T: theory or principle underlying the invention
E: conflicting application
D: document cited in the application
L: citation for other reasons

&: member of the same patent family, corresponding document

| | | | |
|---|---|---|---|
| The present search report has been drawn up for all claims | | | |
| Place of search <br> The Hague | Date of completion of the search <br> 09-05-1980 | Examiner <br> HENRY | |

EPO Form 1503.1 06.78